# DEMANDE DE BREVET EUROPEEN

(11) **EP 0 930 062 A1**
(43) Date de publication de la demande: **21.07.1999**
(21) Numéro de dépôt: 98402942.1
(22) Date de dépôt: 25.11.1998
(51) Int. Cl.: A61K 7/13

(54) **Compositions de teinture des fibres kératiniques contenant des dérivés imidazo-pyridines et procédé**

(30) Priorité: 16.12.1997 FR 9715946
(71) Demandeur: L'OREAL, 75008 Paris (FR)
(72) Inventeur: Terranova, Eric, 92270 Bois Colombes (FR); Fadli, Aziz, 77500 Chelles (FR); Lagrange, Alain, 77770 Coupvray (FR)
(74) Mandataire: Goulard, Sophie

(57) **Abrégé**

L'invention a pour objet une composition pour la teinture d'oxydation des fibres kératiniques contenant au moins un dérivé imidazo-pyridine à titre de coupleur et au moins une base d'oxydation.

L'invention a également pour objet l'utilisation de dérivés imidazo-pyridines à titre de coupleur pour la teinture d'oxydation des fibres kératiniques en association avec au moins une base d'oxydation, ainsi que les procédés de teinture les mettant en oeuvre.

## Description

L'invention a pour objet une composition pour la teinture d'oxydation des fibres kératiniques contenant au moins un dérivé imidazo-pyridine à titre de coupleur et au moins une base d'oxydation.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques. Il est également connu selon la demande de brevet DE-A-4009097 des coupleurs de la famille des imidazo[1,2-a] pyridin-2(3H)-one.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est-à-dire permettre d'obtenir des écarts de coloration les plus faibles possible tout au long d'une même fibre kératinique, qui peut être en effet différemment sensibilisée (i.e. abîmée) entre sa pointe et sa racine.

Or, la demanderesse vient maintenant de découvrir qu'il est possible d'obtenir de nouvelles teintures puissantes, peu sélectives et particulièrement résistantes, capables d'engendrer des colorations intenses dans des nuances variées, en utilisant des dérivés imidazo-pyridines particuliers.

Cette découverte est à la base de la présente invention.

L'invention a donc pour objet une composition pour la teinture d'oxydation des fibres kératiniques et en particulier des fibres kératiniques humaines telles que les cheveux, caractérisée par le fait qu'elle comprend, dans un milieu approprié pour la teinture :
- à titre de coupleur, au moins un dérivé imidazo-pyridine de formule (I) suivante, et/ou au moins l'un de ses sels d'addition avec un acide : 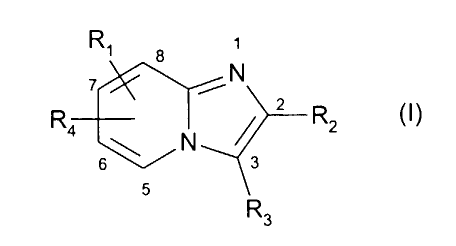 dans laquelle :
   - R₁ représente un atome d'hydrogène, un radical alkyle en C₁-C₄, un radical monohydroxyalkyle en C₁-C₄, un radical polyhydroxyalkyle en C₂-C₄, un radical alkyl (C₁-C₄) amino, un radical dialkyl (C₁-C₄) amino, un radical dialkyl (C₁-C₄) amino alkyl (C₁-C₄), un radical acyl (C₁-C₄) amino, un radical diacyl (C₁-C₄) amino, un radical alcoxy (C₁-C₄) carbonyl alkyl (C₁-C₄), un atome d'halogène, ou un groupement nitro ;
   - R₂ et R₃, identiques ou différents, représentent un atome d'hydrogène, un radical alkyle en C₁-C₄, un radical monohydroxyalkyle en C₁-C₄, un radical polyhydroxyalkyle en C₂-C₄, un radical alcoxy (C₁-C₄) carbonyl, un radical acyle en C₁-C₄, un atome d'halogène, un radical cyano, un radical cyano alkyle en C₁-C₄, un radical alcynyle en C₂-C₄, ou un groupement N-alkyl (C₁-C₄) amido ;
   - R₄ désigne un groupement hydroxyle ou amino ;
- et au moins une base d'oxydation.

Dans la formule (I) ci-dessus, les groupements alkyle en C₁-C₄ et alcoxy en C₁-C₄ peuvent être linéaires ou ramifiés et parmi les atomes d'halogène, on peut citer le chlore, le brome, l'iode et le fluor.

Dans les composés de formule (I) ci-dessus, R₁ peut être de préférence un atome d'hydrogène, un radical alkyle en C₁-C₄, un radical monohydroxyalkyle en C₁-C₄, un radical polyhydroxyalkyle en C₂-C₄, un radical alkyl (C₁-C₄) amino, un radical dialkyl (C₁-C₄) amino, un radical dialkyl (C₁-C₄) amino alkyl (C₁-C₄), un radical acyl (C₁-C₄) amino , un radical diacyl (C₁-C₄) amino , ou un radical alcoxy(C₁-C₄) carbonyl alkyl(C₁-C₄) ;
R₂ et R₃, indifféremment l'un de l'autre, peuvent être de préférence un atome d'hydrogène, un radical alkyle en C₁-C₄, un radical monohydroxyalkyle en C₁-C₄, un radical polyhydroxyalkyle en C₂-C₄, un radical alcoxy (C₁-C₄) carbonyl, un radical acyle en C₁-C₄, ou un groupement N-alkyl (C₁-C₄) amido.

Les composés de formule (I) utilisables à titre de coupleur dans la composition tinctoriale conforme à l'invention sont des composés connus, dont les modes de préparation sont par exemple décrits dans les ouvrages suivants :
- H. L. Blewitt, "Special topics in heterocyclic chemistry", édité par A. Weissberger et E.C. Taylor, édition Interscience, New York, 1977, page 117 ;
- A. E. Tschitschibabin, Berichte, 58, 1704, 1925 ;
- J.C. Teulade & Col., Eur. J. Med. Chem., 13(3), 271, 1978 ;
- W. W. Paudler et H.L. Blewitt, J. Org. Chem., 30, 4081, 1965 ;
- Barton D., Ollis W; D. , "Comprehensive Organic Chemistry", vol 4, page 400-410, édition Pergamon Press ;
- Fusco R., "The Chemistry of Heterocyclic Compounds, Pyrazoles, Pyrazolines, Pyrazolidines, Indazoles and condensed rings", 1967, édité par R. H. Wiley N. Y. ;
- Katritsky A., "Comprehensive heterocyclic chemistry", édition Elsevier.

Les colorations obtenues avec la composition de teinture conforme à l'invention, sont de nuances variées, puissantes, peu sélectives et présentent d'excellentes propriétés de résistance à la fois vis-à-vis des agents atmosphériques tels que la lumière et les intempéries et vis-à-vis de la transpiration et des différents traitements que peuvent subir les cheveux (shampooings, déformations permanentes).

Parmi les dérivés imidazo-pyridines de formule (I) ci-dessus, utilisables à titre de coupleurs dans la composition tinctoriale conforme à l'invention, on peut notamment citer :
- la 8-hydroxy imidazo[1,2-a] pyridine ;
- la 8-hydroxy 2-méthyl imidazo[1,2-a] pyridine ;
- la 8-hydroxy 2,3-diméthyl imidazo[1,2-a] pyridine ;
- la 8-hydroxy 6-chloro 2-méthyl imidazo[1,2-a] pyridine ;
- la 8-hydroxy 3-hydroxyméthyl 2-méthyl imidazo[1,2-a] pyridine ;
- la 8-hydroxy 7-diméthylamino 2-méthyl imidazo[1,2-a] pyridine ;
- le (8-hydroxy 2-méthyl imidazo[1,2-a] pyridin-3-yl)-acétonitrile ;
- le 8-hydroxy-imidazo[1,2-a] pyridin-2,3-acide dicarboxylique bis-éthylamide ;
- le diester éthylique du 8-hydroxy-imidazo[1,2-a] pyridin-2,3-acide dicarboxylique ;
- la 8-amino imidazo[1,2-a] pyridine ;
- la 2-méthyl 8-amino imidazo[1,2-a] pyridine ;
- la 2,3-diméthyl 8-amino imidazo[1,2-a] pyridine ;
- la 2-méthyl 3-hydroxyméthyl 8-amino imidazo[1,2-a] pyridine ;
- la 2,7-diméthyl 8-amino imidazo[1,2-a] pyridine ;
- l'ester éthylique du 7-méthyl 8-amino imidazo[1,2-a] pyridin-2-acide carboxylique ;
- l'ester éthylique du 8-amino imidazo[1,2-a] pyridin-2-acide carboxylique ;
- l'ester éthylique du 3-bromo 7-méthyl 8-amino imidazo[1,2-a] pyridin-2-acide carboxylique ;
- la 2-méthyl 3-prop-2-ynyl 8-amino imidazo[1,2-a] pyridine ;
- la 6-bromo 7-N-éthyl amino imidazo[1,2-a] pyridine ;
- la 6-amino imidazo[1,2-a] pyridine ;
- l'ester éthylique du 6-amino imidazo[1,2-a] pyridin-2-acide carboxylique ;
- l'ester éthylique du 5-méthyl 6-amino imidazo[1,2-a] pyridin-2-acide carboxylique ;
- l'ester éthylique du 6-amino 7-méthyl imidazo[1,2-a] pyridin-2-acide carboxylique ;
- le diester éthylique du 6-amino imidazo[1,2-a] pyridin-2,3-diacide carboxylique ;
- la 5-amino imidazo[1,2-a] pyridine ;
- la 3-méthyl 5-amino imidazo[1,2-a] pyridine ;
- la 2-méthyl 5-amino imidazo[1,2-a] pyridine ;
- la 2,3-diméthyl 5-amino imidazo[1,2-a] pyridine ;
- l'ester éthylique du 2-méthyl 5-amino imidazo[1,2-a] pyridin-3-acide carboxylique ;
- l'ester éthylique du 5-amino imidazo[1,2-a] pyridin-3-acide carboxylique ;
- la 3-acétyl 5-amino imidazo[1,2-a] pyridine ;
- la 2-méthyl 3-acétyl 5-amino imidazo[1,2-a] pyridine ;
et leurs sels d'addition avec un acide.

Le ou les dérivés imidazo-pyridines de formule (I) représentent de préférence de 0,0005 à 12 % en poids environ du poids total de la composition tinctoriale conforme à l'invention, et encore plus préférentiellement de 0,005 à 6 % en poids environ de ce poids.

La nature de la ou des bases d'oxydation pouvant être utilisées dans la composition tinctoriale selon l'invention n'est pas critique. Cette ou ces bases d'oxydation sont de préférence choisies parmi les paraphénylènediamines, les bis-phénylalkylènediamines, les para-aminophénols, les ortho-aminophénols, les bases hétérocycliques, et leurs sels d'addition avec un acide.

Parmi les paraphénylènediamines utilisables à titre de bases d'oxydation dans la composition tinctoriale selon l'invention, on peut notamment citer les composés répondant à la formule (Il) suivante, et leurs sels d'addition avec un acide : 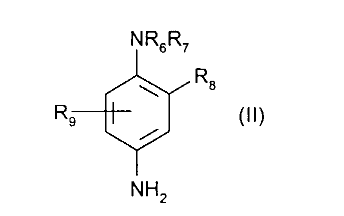 dans laquelle :
- R₆ représente un atome d'hydrogène, un radical alkyle en C₁-C₄, monohydroxyalkyle en C₁-C₄, polyhydroxyalkyle en C₂-C₄ ou alcoxy(C₁-C₄)alkyle(C₁-C₄);
- R₇ représente un atome d'hydrogène, un radical alkyle en C₁-C₄, monohydroxyalkyle en C₁-C₄ ou polyhydroxyalkyle en C₂-C₄ ;
- R₈ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄ ;
- R₉ représente un atome d'hydrogène ou un radical alkyle en C₁-C₄.

Dans la formule (Il) ci-dessus, et lorsque R₈ est différent d'un atome d'hydrogène, alors R₆ et R₇ représentent de préférence un atome d'hydrogène et R₈ est de préférence identique à R₉, et lorsque R₈ représente un atome d'halogène, alors R₆, R₇ et R₉ représentent de préférence un atome d'hydrogène.

Parmi les paraphénylènediamines de formule (Il) ci-dessus, on peut plus particulièrement citer la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, le 4-amino 1-(β-méthoxyéthyl) amino benzène, la 2-chloro paraphénylènediamine, la 2-n-propyl paraphénylènediamine, et leurs sels d'addition avec un acide.

Parmi les bis-phénylalkylènediamines utilisables à titre de bases d'oxydation dans la composition tinctoriale selon l'invention, on peut notamment citer les composés répondant à la formule (III) suivante, et leurs sels d'addition avec un acide : 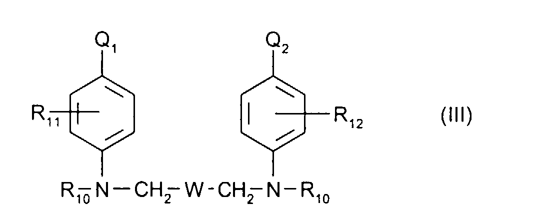 dans laquelle :
- Q₁ et Q₂, identiques ou différents, représentent un radical hydroxyle ou NHR₁₃ dans lequel R₁₃ représente un atome d'hydrogène ou un radical alkyle en C₁-C₄ ;
- R₁₀ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué ;
- R₁₁ et R₁₂, identiques ou différents, représentent un atome d'hydrogène ou d'halogène ou un radical alkyle en C₁-C₄ ;
- W représente un radical pris dans le groupe constitué par les radicaux suivants : 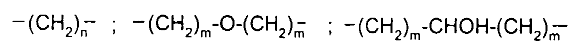 et 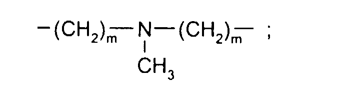 dans lesquels n est un nombre entier compris entre 0 et 8 inclusivement et m est un nombre entier compris entre 0 et 4 inclusivement.

Parmi les bis-phénylalkylènediamines de formules (III) ci-dessus, on peut plus particulièrement citer le N,N'-bis-(β-hydroxyéthyl) N,N'-bis-(4'-aminophényl) 1,3-diamino 2-propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylène-diamine, la N,N'-bis-(éthyl) N,N'-bis-(4'-amino, 3'-méthylphényl) éthylènediamine, et leurs sels d'addition avec un acide.

Parmi ces bis-phénylalkylènediamines de formule (III), le N,N'-bis-(β-hydroxyéthyl) N,N'-bis-(4'-aminophényl) 1,3-diamino 2-propanol ou l'un de ses sels d'addition avec un acide sont particulièrement préférés.

Parmi les para-aminophénols utilisables à titre de bases d'oxydation dans la composition tinctoriale selon l'invention, on peut notamment citer les composés répondant à la formule (IV) suivante, et leurs sels d'addition avec un acide : 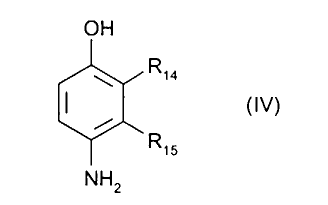 dans laquelle :
- R₁₄ représente un atome d'hydrogène, un radical alkyle en C₁-C₄, monohydroxyalkyle en C₁-C₄, alcoxy(C₁-C₄)alkyle(C₁-C₄) ou aminoalkyle en C₁-C₄ ;
- R₁₅ représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄);
étant entendu qu'au moins un des radicaux R₁₃ et R₁₄ représente un atome d'hydrogène.

Parmi les para-aminophénols de formule (IV) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les ortho-aminophénols utilisables à titre de bases d'oxydation dans la composition tinctoriale selon l'invention, on peut notamment citer le 2-amino phénol, le 2-amino 1-hydroxy 5-méthyl benzène, le 2-amino 1-hydroxy 6-méthyl benzène, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine; la 2,5-diméthyl pyrazolo-[1,5-a]-pyrimidine-3,7-diamine; la pyrazolo-[1,5-a]-pyrimidine-3,5-diamine ; la 2,7-diméthyl pyrazolo-[1,5-a]-pyrimidine-3,5-diamine; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-Amino-pyrazolo[1,5-a]pyrimidin-7-yl)-(2-hydroxy-éthyl)-amino]-éthanol, le 2-[(7-Amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Selon l'invention, la ou les bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale selon l'invention peut également renfermer un ou plusieurs coupleurs additionnels différents des dérivés imidazo-pyridines de formule (I) ci-dessus et/ou un ou plusieurs colorants directs de façon à faire varier ou enrichir en reflets les nuances obtenues avec les bases d'oxydation.

Les coupleurs additionnels utilisables dans la composition selon l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, et leurs sels d'addition avec un acide.

Ces coupleurs peuvent notamment être choisis parmi le 2-méthyl 5-amino phénol, le 5-N-(β-hydroxyéthyl)amino 2-méthyl phénol, le 3-amino phénol, le 1 ,3-dihydroxybenzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthyl)amino 1-méthoxy benzène, le 1,3-diamino benzène, le 1,3-bis-(2,4-diaminophénoxy) propane, le sésamol, l'α-naphtol, le 6-hydroxy indole, le 4-hydroxyindole, le 4-hydroxy N-méthyl indole, la 6-hydroxy indoline, et leurs sels d'addition avec un acide.

Lorsqu'ils sont présents, ces coupleurs additionnels représentent de préférence de 0,0005 à 5 % en poids environ du poids total de la composition tinctoriale, et encore plus préférentiellement de 0,005 à 3 % en poids environ de ce poids.

D'une manière générale, les sels d'addition avec un acide utilisables dans le cadre des compositions tinctoriales de l'invention (composés de formule (I), bases d'oxydation et coupleurs additionnels) sont notamment choisis parmi les chlorhydrates, les bromhydrates, les sulfates et les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol, le glycérol, les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 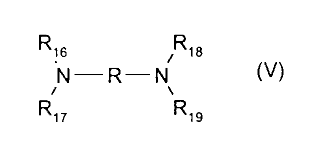 dans laquelle R est un reste propylène éventuellement substitué par un groupement hydroxyle ou un radical alkyle en C₁-C₄ ; R₁₆, R₁₇, R₁₈ et R₁₉, identiques ou différents, représentent un atome d'hydrogène, un radical alkyle en C₁-C₄ ou hydroxyalkyle en C₁-C₄.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet l'utilisation des dérivés imidazo-pyridines de formule (I) ci-dessus, à titre de coupleur, en association avec au moins une base d'oxydation, pour la teinture d'oxydation des fibres kératiniques et en particulier des fibres kératiniques humaines telles que les cheveux.

Un autre objet de l'invention est un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES D'APPLICATION

### EXEMPLES 1 A 5 DE TEINTURE EN MILIEU ALCALIN

On a préparé les compositions tinctoriales conformes à l'invention suivantes :
- 8-hydroxy imidazo[1,2-a] pyridine (coupleur de formule (I)) 0,003 mole
- Base d'oxydation 0,003 mole
- Alcool oléique polyglycérolé à 2 moles de glycérol 4,0 g
- Alcool oléique polyglycérolé à 4 moles de glycérol à 78 % de matières actives (M.A.) 5,69 g M.A.
- Acide oléique 3,0 g
- Amine oléique à 2 moles d'oxyde d'éthylène vendue sous la dénomination commerciale ETHOMEEN O12 ® par la société AKZO 7,0 g
- Laurylamino succinamate de diéthylaminopropyle, sel de sodium à 55 % de M.A. 3,0 g M.A.
- Alcool oléique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium à en solution aqueuse à 35 % de M.A. 0,455 g M.A.
- Acétate d'ammonium 0,8 g
- Antioxydant, séquestrant q.s.
- Parfum, conservateur q.s.
- Ammoniaque à 20 % de NH₃ 10,0 g
- Eau déminéralisée q.s.p. 100 g

Ces compositions ont été préparées avec les bases d'oxydation **B** suivantes :
**B1 :** Paraphénylènediamine
**B2:** Paratoluylènediamine, sulfate
**B3 :** 2-n-propyl paraphénylènediamine
**B4 :** Para-aminophénol
**B5 :** 4-amino 3-méthyl phénol

Au moment de l'emploi, chaque composition tinctoriale décrite ci-dessus a été mélangée poids pour poids avec une solution de peroxyde d'hydrogène à 20 volumes (6 % en poids).

Chaque mélange obtenu a été appliqué pendant 30 minutes, sur des mèches de cheveux gris naturels à 90 % de blancs, à raison de 28 g pour 3 g de cheveux. Après rinçage, lavage avec un shampooing standard et séchage, les mèches ont été teintes dans les nuances figurant dans le tableau I ci-dessous :

**Tableau I**

| **Exemple** | **Base d'oxydation** | **Nuance** |
|---|---|---|
| 1 | **B1** | Blond irisé cendré |
| 2 | **B2** | Blond beige doré |
| 3 | **B3** | Blond gris |
| 4 | **B4** | Blond clair doré irisé |
| 5 | **B5** | Blond clair irisé à reflet doré |

### EXEMPLES 6 A 17 DE TEINTURE EN MILIEU BASIQUE

On a préparé les compositions tinctoriales conformes à l'invention suivantes :
- Coupleur de formule (I) 0,003 mole
- Base d'oxydation 0,003 mole
- Alcool éthylique à 96° 18 g
- Métabisulfite de sodium en solution aqueuse à 35% 0,68 g
- Sel pentasodique de l'acide diéthylène triaminopentacétique 1,1 g
- Ammoniaque à 20% de NH₃ 10 g
- Eau déminéralisée q.s.p. 100 g

Ces compositions ont été préparées avec les coupleurs **C** de formule (I) et les bases d'oxydation **B** suivantes :
**C1 :** 5-amino imidazo[1,2-a] pyridine, 1 HCI
**C2 :** 8-amino imidazo[1,2-a] pyridine, 2 HCI

**B1 :** Paraphénylènediamine
**B4 :** Para-aminophénol
**B5 :** 4-amino 3-méthyl phénol
**B6** : Paratoluylènediamine
**B7** : 4,5-diamino 1,3-diméthyl pyrazole, 2 HCI
**B8 :** Pyrazolo-[1,5-a]-pyrimidine-3,7-diamine, 2 HCI

Au moment de l'emploi, chacune des compositions tinctoriales décrites ci-dessus a été mélangée poids pour poids avec une solution de peroxyde d'hydrogène à 20 volumes (6 % en poids).

Chacun des mélanges obtenus présentait un pH d'environ 9,8 ± 0,2 et a été appliqué pendant 30 minutes, sur des mèches de cheveux gris naturels à 90 % de blancs, à raison de 28 g pour 3 g de cheveux. Après rinçage, lavage avec un shampooing standard et séchage, les mèches ont été teintes dans les nuances figurant dans le tableau Il ci-dessous :

**Tableau II**

| **Exemple** | **Coupleur** | **Base** | **Nuance** |
|---|---|---|---|
| 6 | **C1** | **B1** | Châtain irisé rouge |
| 7 | **C1** | **B6** | Châtain irisé rouge |
| 8 | **C1** | **B4** | Blond clair doré |
| 9 | **C1** | **B7** | Pourpre bleuté |
| 10 | **C1** | **B8** | Rouge |
| 11 | **C1** | **B5** | Blond clair beige irisé |
| 12 | **C2** | **B1** | Blond foncé irisé rouge |
| 13 | **C2** | **B6** | Blond irisé rouge |
| 14 | **C2** | **B4** | Blond clair doré cuivré |
| 15 | **C2** | **B7** | Blond clair doré mat |
| 16 | **C2** | **B8** | Blond foncé doré acajou |
| 17 | **C2** | **B5** | Blond clair irisé |

### EXEMPLES 18 A 29 DE TEINTURE EN MILIEU NEUTRE :

On a préparé les compositions tinctoriales conformes à l'invention suivantes :
- Coupleur de formule (I) 0,003 mole
- Base d'oxydation 0,003 mole
- Alcool éthylique à 96° 18 g
- Métabisulfite de sodium en solution aqueuse à 35% 0,68 g
- Sel pentasodique de l'acide diéthylène triaminopentacétique 1,1 g
- Ammoniaque à 20% de NH₃ 10 g
- Tampon phosphate K₂HPO₄/ KH₂PO₄ (1,5M / 1M ) 10 g
- Eau déminéralisée q.s.p. 100 g

Ces compositions ont été préparées avec les coupleurs **C** de formule (I) et les bases d'oxydation **B** suivantes :
**C1 :** 5-amino imidazo[1,2-a] pyridine, 1 HCI
**C2 :** 8-amino imidazo[1,2-a] pyridine, 2 HCI

**B1 :** Paraphénylènediamine
**B4 :** Para-aminophénol
**B5 :** 4-amino 3-méthyl phénol
**B6** : Paratoluylènediamine
**B7** : 4,5-diamino 1,3-diméthyl pyrazole, 2 HCI
**B8 :** Pyrazolo-[1,5-a]-pyrimidine-3,7-diamine, 2 HCI

Chacune des compositions tinctoriales a été appliquée sur des mèches de cheveux gris naturels à 90 % de blancs selon le procédé de teinture décrit ci-dessus pour les exemples 6 à 17. Chacun des mélanges obtenu présentait un pH compris entre 6 et 7 environ.

Les cheveux ont été teints dans une nuance figurant dans le tableau III ci-après :

**Tableau III**

| **Exemple** | **Coupleur** | **Base** | **Nuance** |
|---|---|---|---|
| 18 | **C1** | **B1** | Brun violine rabattu |
| 19 | **C1** | **B6** | Châtain doré acajou cendré |
| 20 | **C1** | **B4** | Blond clair doré cuivré |
| 21 | **C1** | **B7** | Gris bleuté rosé |
| 22 | **C1** | **B8** | Acajou cendré rabattu |
| 23 | **C1** | **B5** | Blond clair doré légèrement mat |
| 24 | **C2** | **B1** | Châtain cendré violine |
| 25 | **C2** | **B6** | Châtain cendré violine rabattu |
| 26 | **C2** | **B4** | Blond clair cuivré doré |
| 27 | **C2** | **B7** | Blond clair cendré irisé |
| 28 | **C2** | **B8** | Châtain violine |
| 29 | **C2** | **B5** | Blond clair irisé doré |

## Revendications

1. Composition pour la teinture d'oxydation des fibres kératiniques et en particulier des fibres kératiniques humaines telles que les cheveux, caractérisée par le fait qu'elle comprend, dans un milieu approprié pour la teinture :
- à titre de coupleur, au moins un dérivé imidazo-pyridine de formule (I) suivante, et/ou au moins l'un de ses sels d'addition avec un acide : 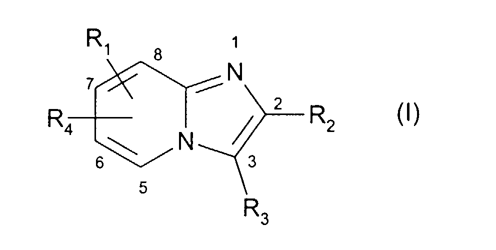 dans laquelle :
- R₁ représente un atome d'hydrogène, un radical alkyle en C₁-C₄, un radical monohydroxyalkyle en C₁-C₄, un radical polyhydroxyalkyle en C₂-C₄, un radical alkyl (C₁-C₄) amino, un radical dialkyl (C₁-C₄) amino, un radical dialkyl (C₁-C₄) amino alkyl (C₁-C₄), un radical acyl (C₁-C₄) amino, un radical diacyl (C₁-C₄) amino, un radical alcoxy (C₁-C₄) carbonyl alkyl (C₁-C₄), un atome d'halogène, ou un groupement nitro ;
- R₂ et R₃, identiques ou différents, représentent un atome d'hydrogène, un radical alkyle en C₁-C₄, un radical monohydroxyalkyle en C₁-C₄, un radical polyhydroxyalkyle en C₂-C₄, un radical alcoxy (C₁-C₄) carbonyl, un radical acyle en C₁-C₄, un atome d'halogène, un radical cyano, un radical cyano alkyle en C₁-C₄, un radical alcynyle en C₂-C₄, ou un groupement N-alkyl (C₁-C₄) amido ;
- R₄ désigne un groupement hydroxyle ou amino ;
- et au moins une base d'oxydation.

2. Composition selon la revendication 1, caractérisée par le fait que les composés de formule (I) sont choisis parmi :
- la 8-hydroxy imidazo[1,2-a] pyridine ;
- la 8-hydroxy 2-méthyl imidazo[1,2-a] pyridine ;
- la 8-hydroxy 2,3-diméthyl imidazo[1,2-a] pyridine ;
- la 8-hydroxy 6-chloro 2-méthyl imidazo[1,2-a] pyridine ;
- la 8-hydroxy 3-hydroxyméthyl 2-méthyl imidazo[1,2-a] pyridine ;
- la 8-hydroxy 7-diméthylamino 2-méthyl imidazo[1,2-a] pyridine ;
- le (8-hydroxy 2-méthyl imidazo[1,2-a] pyridin-3-yl)-acétonitrile ;
- le 8-hydroxy-imidazo[1,2-a] pyridin-2,3-acide dicarboxylique bis-éthylamide ;
- le diester éthylique du 8-hydroxy-imidazo[1,2-a] pyridin-2,3-acide dicarboxylique ;
- la 8-amino imidazo[1,2-a] pyridine ;
- la 2-méthyl 8-amino imidazo[1,2-a] pyridine ;
- la 2,3-diméthyl 8-amino imidazo[1,2-a] pyridine ;
- la 2-méthyl 3-hydroxyméthyl 8-amino imidazo[1,2-a] pyridine ;
- la 2,7-diméthyl 8-amino imidazo[1,2-a] pyridine ;
- l'ester éthylique du 7-méthyl 8-amino imidazo[1,2-a] pyridin-2-acide carboxylique ;
- l'ester éthylique du 8-amino imidazo[1,2-a] pyridin-2-acide carboxylique ;
- l'ester éthylique du 3-bromo 7-méthyl 8-amino imidazo[1,2-a] pyridin-2-acide carboxylique ;
- la 2-méthyl 3-prop-2-ynyl 8-amino imidazo[1,2-a] pyridine ;
- la 6-bromo 7-N-éthyl amino imidazo[1,2-a] pyridine ;
- la 6-amino imidazo[1,2-a] pyridine ;
- l'ester éthylique du 6-amino imidazo[1,2-a] pyridin-2-acide carboxylique ;
- l'ester éthylique du 5-méthyl 6-amino imidazo[1,2-a] pyridin-2-acide carboxylique ;
- l'ester éthylique du 6-amino 7-méthyl imidazo[1,2-a] pyridin-2-acide carboxylique ;
- le diester éthylique du 6-amino imidazo[1,2-a] pyridin-2,3-diacide carboxylique ;
- la 5-amino imidazo[1,2-a] pyridine ;
- la 3-méthyl 5-amino imidazo[1,2-a] pyridine ;
- la 2-méthyl 5-amino imidazo[1,2-a] pyridine ;
- la 2,3-diméthyl 5-amino imidazo[1,2-a] pyridine ;
- l'ester éthylique du 2-méthyl 5-amino imidazo[1,2-a] pyridin-3-acide carboxylique ;
- l'ester éthylique du 5-amino imidazo[1,2-a] pyridin-3-acide carboxylique ;
- la 3-acétyl 5-amino imidazo[1,2-a] pyridine ;
- la 2-méthyl 3-acétyl 5-amino imidazo[1,2-a] pyridine ;
et leurs sels d'addition avec un acide.

3. Composition selon la revendication 1 ou 2, caractérisée par le fait que le ou les composés de formule (I) représentent de 0,0005 à 12 % en poids du poids total de la composition tinctoriale.

4. Composition selon la revendication 3, caractérisée par le fait que le ou composés de formule (I) représentent de 0,005 à 6 % en poids du poids total de la composition tinctoriale.

5. Composition selon l'une quelconque des revendications précédentes, caractérisée par le fait que la ou les bases d'oxydation sont choisies parmi les paraphénylènediamines, les bis-phénylalkylènediamines, les para-aminophénols, les ortho-aminophénols, les bases hétérocycliques, et leurs sels d'addition avec un acide.

6. Composition selon l'une quelconque des revendications précédentes, caractérisée par le fait que la ou les bases d'oxydation représentent de 0,0005 à 12 % en poids du poids total de la composition tinctoriale.

7. Composition selon la revendication 6, caractérisée par le fait que la ou les bases d'oxydation représentent de 0,005 à 6 % en poids du poids total de la composition tinctoriale.

8. Composition selon l'une quelconque des revendications précédentes, caractérisée par le fait que les sels d'addition avec un acide sont choisis parmi les chlorhydrates, les bromhydrates, les sulfates et les tartrates, les lactates et les acétates.

9. Composition selon l'une quelconque des revendications précédentes, caractérisée par le fait qu'elle renferme un ou plusieurs coupleurs additionnels différents des composés de formule (I) tels que définis à la revendication 1 ou 2 et/ou un ou plusieurs colorants directs.

10. Composition selon l'une quelconque des revendications précédentes, caractérisée par le fait que le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

11. Composition selon l'une quelconque des revendications précédentes, caractérisée par le fait qu'elle présente un pH compris entre 3 et 12.

12. Composition selon l'une quelconque des revendications précédentes, caractérisée par le fait qu'elle se présente sous forme de liquides, de crèmes, de gels, ou sous toute autre forme appropriée pour réaliser une teinture des fibres kératiniques, et notamment des cheveux humains.

13. Utilisation des composés de formule (I) tels que définis à l'une quelconque des revendications 1 à 4, à titre de coupleurs dans des compositions pour la teinture d'oxydation des fibres kératiniques et en particulier des fibres kératiniques humaines telles que les cheveux, en association avec au moins une base d'oxydation.

14. Procédé de teinture d'oxydation des fibres kératiniques et en particulier des fibres kératiniques humaines telles que les cheveux, caractérisé par le fait qu'on applique sur ces fibres au moins une composition tinctoriale telle que définie à l'une quelconque des revendications 1 à 12, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

15. Procédé selon la revendication 14, caractérisé par le fait que l'agent oxydant est choisi parmi le peroxyde d'hydrogène, le peroxyde d'urée, les bromates de métaux alcalins, les persels tels que les perborates et persulfates.

16. Dispositif à plusieurs compartiments, ou "kit" de teinture à plusieurs compartiments, dont un premier compartiment renferme une composition tinctoriale telle que définie à l'une quelconque des revendications 1 à 12 et un second compartiment renferme une composition oxydante.
